# EUROPEAN PATENT APPLICATION

(11) **EP 4 647 046 A2**
(43) Date of publication of application: **12.11.2025**
(21) Application number: 25172305.2
(22) Date of filing: 24.04.2025
(51) Int. Cl.: A61F 2/58

(54) **MODULE FOR CONNECTING GRIPPING TOOLS TO A COUPLING TERMINAL ON AN UPPER LIMB STUMP**

(30) Priority: 09.05.2024 IT 202400010507
(71) Applicant: RO.GA. S.p.A., 94100 Enna (EN) (IT)
(72) Inventor: Colaleo, Stefano Rosario Maria, 94100 Enna (EN) (IT)
(74) Representative: Karaghiosoff, Giorgio Alessandro

(57) **Abstract**

Connection module for coupling a gripping tool to a coupling terminal of a stump, which connection module has a first module part (1) intended to be fixed to said coupling terminal to the stump and a second module part (2) intended to be fixed or connected to a gripping tool forming a one piece part, while said first part and said second part are provided with reciprocal coupling and release members (4, 5), wherein said first and/or said second part have an actuator (8) and release members for controlling said reciprocal coupling. Said control actuator cooperating with an actuation member of said control actuator to activate the reciprocal coupling and release members of said two parts of the connection module.

## Description

### Technical field of the invention

The invention has as its object a module for connecting a gripping tool called an end effector to a coupling terminal to a stump, which connection module has a first module part intended to be fixed to said coupling terminal to the stump and a second module part intended to be fixed or connected as one piece to a gripping tool, while said first part and said second part are provided with removable coupling members provided in a pre-established relative position one with respect to the other.

### Background of the invention

The upper limb can be described as a system whose main effector organ is the hand, thanks to which a person is able to perform an extremely wide variety of functions and skills that are used in the different activities of daily life, work and sports.

Grasping and manipulation are undoubtedly the predominant functions of the hand, however, thanks to its extero-proprioceptive sensitivity, it can be considered as a sensing organ capable among others of protecting from injuries, defining the profiles of objects and perceiving temperature.

The hand is also one of the main and most refined means of expressing the psycho-emotional atmosphere of man, becoming part, in the context of gestures, of the so-called body language.
Considering this extreme complexity of the functions of the hand, it is understandable how over the years great efforts have been made to create a prosthetic hand that best meets the different needs of amputees.

The most frequent cause of upper limb amputation, with an incidence of 77% (Amputees and their prostheses, Artificial limbs 1970, E. J. Davies et al.), is trauma, mainly due to accidents at work, therefore, involving patient still of working age at the time of the injury. Congenital deformities, including their surgical revisions, and diseases account for approximately 23% overall.

The levels of upper limb amputation, excluding the hand, starting from the one that allows the application of an electromechanical hand, are the following:
- transcarpal;
- radiocarpal (disarticulation);
- transradial (distal, proximal, between the first two lengths);
- elbow (disarticulation);
- transhumeral (distal, proximal, between the first two lengths);
- glenohumeral (disarticulation);
- inter-scapular-thoracic.

Innovation in the field of prosthetics has made it possible for many people who have undergone upper limb amputation to continue cultivating their passions or even discover a new one. It is not unusual, in fact, for those who begin a new daily life with an upper limb prosthesis to decide to try new activities or sports, in search of a renewed identity and a source of self-esteem.

Each type of activity requires specific movements, a different weight balance and the use of specific muscles.

At present, removable attachment modules for tools such as end effectors of various types to a terminal coupling to a stump are known. However, the known systems, although functionally effective, often require complex handling that makes the replacement of the end effector, i.e. the gripping tool or similar, relatively complex and time-consuming.

Furthermore, for people who have undergone bilateral amputations of the upper limbs, the known systems do not allow the tool replacement processes to be performed easily and quickly. In fact, the coupling and mutual release mechanisms of the two parts of a module often require the execution of operations that require the use of at least one hand, so in some cases, the tool replacement cannot be performed by the amputee alone. The need to be able to replace the terminal gripping tools easily and quickly lies in the fact that to perform various activities it is necessary to use tools that first allow you to grip the tools needed to carry out the various activities (sports and daily) and then perform, with maximum comfort, the functional movements required to carry out the aforementioned activities.

Document GB109259 discloses a connection module of a gripping tool to a stump coupling terminal having a first module part intended to be fixed to said stump coupling terminal and a second module part intended to be fixed or to be connected integrally to a gripping tool. Said first and said second parts are provided with mutual coupling and uncoupling members. As mutual coupling and uncoupling member an arrangement for securing and releasing the gripping tool is provided in the shape of two studs, set-screws or press buttons set opposite or counter to each other necessitating a hand to be operated in order to couple and uncouple the tool from the coupling terminal to the stump.

### Brief description of the invention

According to a first aspect, an object of the present invention consists in realizing a combination of a aforementioned connection module with at least one gripping tool or at least one end effector, preferably a plurality of different gripping tools or end effectors, that are configured in a specific way each for the execution of at least one specific different activity and of realizing said module in such a way that the two parts of said module, i.e. the one fixed or fixable to the coupling terminal to the stump and the one fixed or made of one piece to a gripping tool or an end effector can be coupled together in a simple, effective and easily operated way both for the connection or mutual engagement and for the detachment or mutual disengagement of said two parts of the module.

According to a further aspect, the present invention aims to create a connection module between a gripping tool and a coupling terminal to a stump, which module can be operated upon coupling or engagement of a gripping tool or of an end effector and/or upon detachment or disengagement of said gripping tool or said end effector without requiring any assistance by means of a hand and/or a gripping tool or an end effector of the other upper limb of the same amputee user or of an assistant.

According to yet another aspect, the invention also provides for a plurality of different gripping tools or end effectors that can be fixed alternately to one another at the same coupling terminal to a stump thanks to a connection module comprising two parts that can be engaged and disengaged from one the other, a first of said two parts being fixed or fixable to the coupling terminal to the stump and the other of said two parts constituting a base for engagement and disengagement from said first part, while each of the different gripping tools and/or different end effectors is provided with its own engagement and disengagement base, all the engagement and disengagement bases being equal to one another and suitable for cooperating with said first part of the connection module.

According to a first embodiment, the invention has as its object a connection module of a gripping tool or a so-called end effector to a coupling terminal to a stump, which connection module has a first module part intended to be fixed to said coupling terminal to the stump and a second module part intended to be fixed or connected as one piece to a gripping tool or to an end effector, while said first part and said second part are provided with reciprocal coupling and release members
and in which said first and/or said second parts have an actuator for controlling said reciprocal coupling and release members alternately from one to the other of said reciprocal coupling and release conditions
and which control actuator cooperates with an actuation member of said control actuator to activate the reciprocal coupling and release members of said two parts of the connection module,
and wherein said actuating member is provided in a predetermined position in a parking cradle of said gripping tool and/or of said end effector and cooperates with said actuator during an insertion stroke of said gripping tool and/or of said end effector into said cradle and/or during a removal stroke of said gripping tool and/or of said end effector from said cradle, automatically determining the actuation of the mutual coupling and uncoupling members respectively,
in the condition of mutual uncoupling of said first from said second part of the connection module, when with the movement of the stump said module with the corresponding gripping tool and/or with the corresponding end effector are brought into a parking condition inside said parking housing and therefore freeing the first part of the module from the second part of the module,
and in the condition of mutual coupling of the said first part with the said second part of the connection module, when with the movement of the stump the said first part of the module is brought into a position of readiness for coupling to the said second part of the said module which is provided as the base of a pre-established gripping tool or of a pre-established end effector and subsequently the said first part of the module and the said second part of the module, with the corresponding gripping tool and/or with the corresponding end effector, are extracted along a pre-established path from the said parking condition inside the said parking housing, extracting from the said parking cradle and therefore making the said connection module usable with the corresponding gripping tool or with the corresponding end effector.

Different embodiments are possible for the said mutual coupling and uncoupling devices of the two parts of the said connection module, of the said actuator and of the said actuation device, as well as of the parking cradle or cradles of the ensembles consisting of a second module part and a gripping tool and/or an end effector.

The invention provides a preferred, non-limiting embodiment, in which the control actuator is constituted by a pusher provided with a sliding head at its end and with which the said pusher protrudes externally from a perimeter face of the casing of the said second module part,
while said pusher is supported sliding in both directions in a direction transverse to said perimeter face and
while said actuating members are constituted by a shaped cam surface with a predetermined position with respect to the parking position of the gripping tool and/or the end effector in said parking cradle
and with a shape of the surface profile of said cam configured such that upon insertion of the gripping tool and/or of the end effector into said parking cradle and upon extraction of the gripping tool and/or of the end effector, said sliding head slides along said shaped cam surface controlling the actuator controlling the coupling and uncoupling members, in the corresponding coupling condition of said first part to said second part of the connection module or alternatively in the corresponding uncoupling condition of said first part from said second part of said connection module respectively by means of executing the insertion path of said gripping tool and/or of said end effector into said housing cradle and by means of executing the extraction path of said gripping tool and/or of said end effector from said parking cradle.

In a preferred and non-limiting embodiment, said pusher is stably stressed by one or more elastic elements in a position of end of stroke and of greatest protrusion outwards from said face of the perimeter wall of the said second and/or of said first part of said connection module and the shaped surface of the cam has a shape such that in the terminal parking position of the gripping tool and/or of the end effector in said parking cradle, said pusher is moved against the action of the said elastic element(s) in a position of end of stroke of less protrusion outwards from said face of said perimeter wall of the said mantle wall and while during the extraction path of the gripping tool and/or of the end effector the shape of said cam surface is such that said pusher is progressively released and returns to the aforementioned end of stroke position, of maximum protrusion outwards from said face of the perimeter surface of the said mantle wall.

In a preferred, but not limiting, embodiment, the mutual coupling and uncoupling members of said first part and said second part of the connection module are respectively constituted by a central coupling pin with an enlarged head protruding from a head side of said first part of the connection module in the direction of said second part of the connection module and which coupling pin is preferably oriented in the direction of mutual coupling and uncoupling of said two parts,
while the said second part has a seat for engaging the said coupling pin which is in a position axially coinciding with the said coupling pin and which has at least one coupling tooth with the enlarged head of the said coupling pin, which coupling tooth is movable along a coupling/uncoupling stroke alternatively in a position radially spaced from the perimeter of the said enlarged head of the said coupling pin and in a position radially close to the said coupling pin and axially adjacent to the radial shoulder formed by the said enlarged head and posteriorly to the said radial shoulder with reference to the direction of engagement of the said coupling pin in the said coupling seat of the said second part,
said engagement tooth being controlled to execute said engagement/release stroke by said control actuator organ and by said actuation organ, or by said pusher.

In a preferred embodiment, said first part and said second part have end-of-stroke abutment surfaces facing each other and which abutment surfaces have coinciding rings of notches for relative angular positioning of said first and second parts with respect to each other.

According to yet another possible embodiment that is envisaged in combination with one or more of the previous features or embodiments, the connection module has a rotationally symmetrical shape, the coupling and uncoupling direction of the said two parts being parallel to the central axis of the connection module.

With reference to the embodiments described above, the aforementioned, rotationally symmetrical embodiment provides that the axis of symmetry coincides with the coupling pin and with the corresponding coupling seat of the said second part, while the said rings of reciprocal angular positioning notches are made up of two concentric circular annular rings of axial teeth, preferably with a rounded or substantially sinusoidal profile.

In one embodiment, one of the said rings of axial angular positioning notches is designed to be mountable and removable from the corresponding second part of the connection module, optionally by means of a bayonet-type interlocking system.

Still according to a further feature which may be provided in combination with one or more of the preceding embodiments, said engagement tooth is constituted by the peripheral edge of a circular hole provided in a frame, which hole has a diameter slightly larger than the largest diameter of the enlarged head of the engagement pin and which frame is mounted so as to slide in the direction of a diametral axis of the engagement hole, preferably parallel to at least one side of said frame, which axis is parallel with the sliding axis of said pusher and which pusher controls the movement of said frame at least in one direction, while the movement in the opposite direction is determined by said elastic elements, and the movement of the frame in the radially outward direction of the pusher being caused by said elastic means while the movement in the opposite direction is caused by said cam surface, and wherein said elastic elements bring the frame into an eccentric position of the hole with respect to the axis of the engagement pin and wherein a part of the peripheral edge of said frame position itself in posteriorly to the annular, radial shoulder of the enlarged head with reference to the direction of engagement of the coupling pin in said coupling seat and while in said position in which the end of stroke pusher having the lowest protrusion outwards from said face of said perimeter wall of the mantle wall, said frame is brought by said pusher into a coaxial position with said enlarged head of the coupling pin.

Still according to an embodiment, at least the enlarged head of the coupling pin has a rotationally symmetrical shape and optionally conically tapered towards the front end with reference to the direction of coupling of the said two module parts with each other and/or of insertion of the coupling pin into the corresponding coupling seat.

The combination of a coupling pin with a conical head whose base radius is greater than the radius of the stem of the coupling pin with a coupling hole with a radius slightly greater than the radius of the base said conical enlarged head and which hole is provided in a sliding support frame sliding, in an axial direction, between a coaxial position with said conical enlarged head and in an eccentric position with said conical head in which said frame is stably stressed by elastic elements, allows for an automatic and stable coupling of the two module parts one with the other, it being possible to release the pin from said hole only by means of an active action of moving the pusher thanks to the shaped cam surface with which action said frame is moved against the action of the elastic means, radially from the eccentric position of the hole with respect to the conical head of the coupling pin to the coaxial position of said hole with respect to said conical head which coaxial position allows the coupling pin to be removed from the coupling seat and therefore the separation of the first from the second part of the module.

With reference to the configuration of the cam-shaped surface, this is expected to coincide with a sector of the side wall of a parking cradle for a gripping tool and/or for an end effector, which sector of the side wall is intended to overlap laterally with the perimeter casing wall of the second part of the connection module and which wall sector is at least partially U-shaped or has two diametrically opposed sides that converge towards each other in the direction of the end-of-stroke insertion position of the gripping tool and/or of the end effector and of the second part of the module associated with them, and while one of the said two side walls of the said U-shaped sector cooperates with the sliding head of the pusher and the other of the said two side walls of the said U-shaped sector cooperates with the face of the perimeter mantle wall of the casing of the said second part of the connection module that is diametrically opposite to the said sliding head of the said pusher, and while the reduction of the distance between the said two side walls corresponds to the displacement stroke of the pusher in the radial direction of the coupling pin and/or of the frame with the coupling hole with which said coupling hole is brought into a coaxial position with the said coupling pin.

In one embodiment, the said parking cradle is constituted by a concave housing which, in correspondence with an open side, presents the said U-shaped wall sector which constitutes the cam-shaped surface.

In one embodiment, the parking cradle is open on two opposite sides parallel to the cross-section plane of the U-shaped wall, and this is provided oriented with its axis in a substantially vertical direction.

In one embodiment, it is possible to provide two or more parking cradles combined together in a single magazine which is intended to accommodate correspondingly two or more gripping tools or two or more end effectors each one configured differently and for the execution of different actions or activities and which two or more gripping tools and/or which two or more end effectors are each one provided with a connection base to a terminal for coupling to a stump which is made as the said second part of the said connection module and is identical for each of the two or more gripping tools and/or for each of the two or more end effectors, while the said coupling terminal is fixed or can be fixed to the said first part of the said connection module, so that it is possible to replace the gripping tool and/or the end effector connected to the terminal for coupling to the stump with any of the tools or end effectors present in the respective cradles of the parking magazines.

Thanks to the above-mentioned features, the advantages of the present invention are evident. In fact, the attachment of a specific gripping tool or a specific end effector to a coupling terminal to a stump of an upper limb does not require any type of action to be performed with the twin limb, but the attachment or detachment of a tool or an end effector to a coupling terminal can be performed directly using the amputated arm.

This provides a notable advantage when the user is a bilateral amputee, i.e. has undergone amputations of both upper limbs. In this case, the user can operate replacements of the gripping tools or end effectors specifically configured for different activities, without needing the help of third parties, but can operate such replacements autonomously.

Thanks to the invention, it is therefore possible for an amputee, even a bilateral amputee, to use tools or end effectors that are configured to grip the instruments necessary to carry out various activities (sports and daily) and subsequently perform, with maximum comfort, the functional movements required to carry out the aforementioned activities. As will be better appear from the following description, said tools, or said end effectors can be configured to allow the upper limb amputee to carry out both sports activities such as gym, basketball, swimming, fishing, etc. and domestic activities (personal hygiene, cooking, eating, etc.).

The various types of combinations of gripping tools and/or of end effectors configured specifically for different purposes and of a fixing base configured as the said second part of the connection module according to one or more of the previously described embodiments can be part of a kit and the various tools or end effectors will be contained within a warehouse where the user can easily insert and remove them from a corresponding parking cradle, at any time, without the need to use the contralateral limb, based on the activity to be performed by means of a pin attachment system. The attachment is unique for all the gripping tools or end effectors, and this allows the use of a single socket for various types of gripping tools end effectors and therefore various uses.

Therefore, thanks to the invention, the objective of allowing all upper limb amputees to improve their quality of life has been achieved since they can again independently perform some activities that they were previously unable to do without these accessories.

Alternatively or in combination with one or more of the embodiments described above, the invention provides a particular gripping tool that is configured in the shape of a hand and that has at the distal end of the fingers a coupling module to a terminal for fixing to a stump, while said hand part is shaped similarly to a hand in gripping position for a gripping handle having a longitudinal dimension which is larger than a transversal dimension relatively to said longitudinal dimension and the position of the thumb being opposed or facing to the position of the remaining fingers so as to form a housing channel, respectively circumferential clamping members of a handle being provided which are positioned at the two opposite lateral ends of the palm of the hand, said clamping members being elastically tightenable and/or being tightenable by tensioning around a handle of a tool.

In one embodiment, said clamping elements are in the form of a band, a cable, a rope or a string, or the like.

According to one embodiment, the clamping elements in the form of a band, a cable, a cord, or a string, or the like each form at least one loop and have a predetermined initial tension, the following configurations being provided alternatively or in combination with each other:
the preventive tension is such that the fixing of a handle occurs by forcing it when it is introduced into the loop;
the preventive tension is such that it is further modified or adjusted with respect to the clamping force by means of a predetermined force, a tensioning/loosening mechanism being provided in combination with at least some of said clamping elements, which can be operated manually or by means of an electric motor.

In the embodiment in which said clamping members are constituted by loops formed by said elastic element which has a pre-established initial tension such that the length of the section of the clamping element which forms the loop is less than a pre-established average dimension of the cross section of a grip handle, said grip handle is inserted into the loop by widening the same by forcing said grip handle at the time of insertion and said elastic element automatically exerts a pre-established clamping force of said grip handle against the palm of the hand.

In the second embodiment, said clamping elements are provided relatively loose and with a length along the loop such that said loop is greater than a predetermined circumferential extension of the section of a handle, the clamping element being tightened with a predetermined force around said handle by means of said tensioning and loosening mechanism which can be operated manually or by means of an electric motor, while the loosening of the clamping element is performed to free the handle from said hand.

In one embodiment, the hand is made hollow at least in the area coinciding with the part of the back of the hand and the cavity is closed towards the outside by a removable cover which is preferably shaped so as to complete the shape of the back of the hand at the opening of said cavity and which has removable coupling elements with corresponding and coinciding elements provided in said cavity.

In one embodiment, said cavity constitutes the housing compartment for the terminal ends of the clamping members and/or the housing compartment for the tensioning and loosening mechanism(s) of said clamping members, as well as for the possible drive motor(s) of said tensioning and loosening members and the related control circuits and/or power batteries.

### Brief description of the drawings

The above characteristics and further characteristics of the invention as well as the advantages obtained with them are the subject of the following description of some preferred but not limiting embodiments illustrated in the attached drawings in which:
Figure 1 shows an exploded perspective view of an embodiment of a connection module according to the present invention.
Figures 2 to 4 show different views of the embodiment according to Figure 1.
Figures 5 to 7 show different views of a parking warehouse for two gripping tools or two end effectors according to an embodiment of the present invention, particularly suitable for personal hygiene care activities.
Figures 8 to 12 show different views of the parking warehouse according to the previous figures in combination with two gripping tools each configured for a different activity relating to personal hygiene care.
Figures 13 to 16 show different views of a further embodiment of the present invention relating to a parking cradle for a personal hygiene tool.
Figures 17 to 20 show different views of the combination of the specific gripping tool with the parking cradle according to the previous figures.
Figures 21 to 25 show different views of a first embodiment of the present invention which provides a transportable box comprising three parking cradles for three different gripping tools intended for sports activities and in which the paths for inserting and extracting the individual gripping tools into and from the corresponding parking cradle are shown.
Figures 26 to 28 show an embodiment of the box according to Figures 21 to 25, in which the parking cradles are made according to an alternative shape inspired by the cradle of the embodiment according to Figures 13 to 16.
Figures 29 to 36 show different views of a gripping tool specifically configured for gripping cutlery or similar objects having handles with a longitudinal dimension greater than the transverse or radial dimension.

### Detailed description of the figures

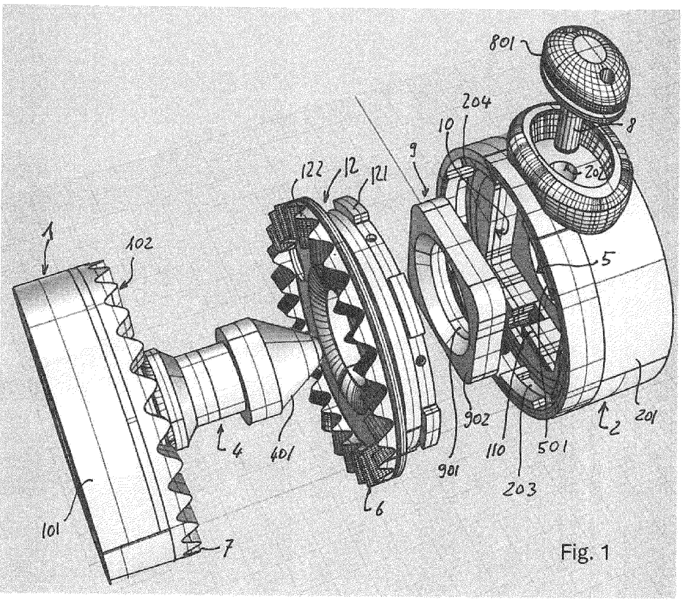

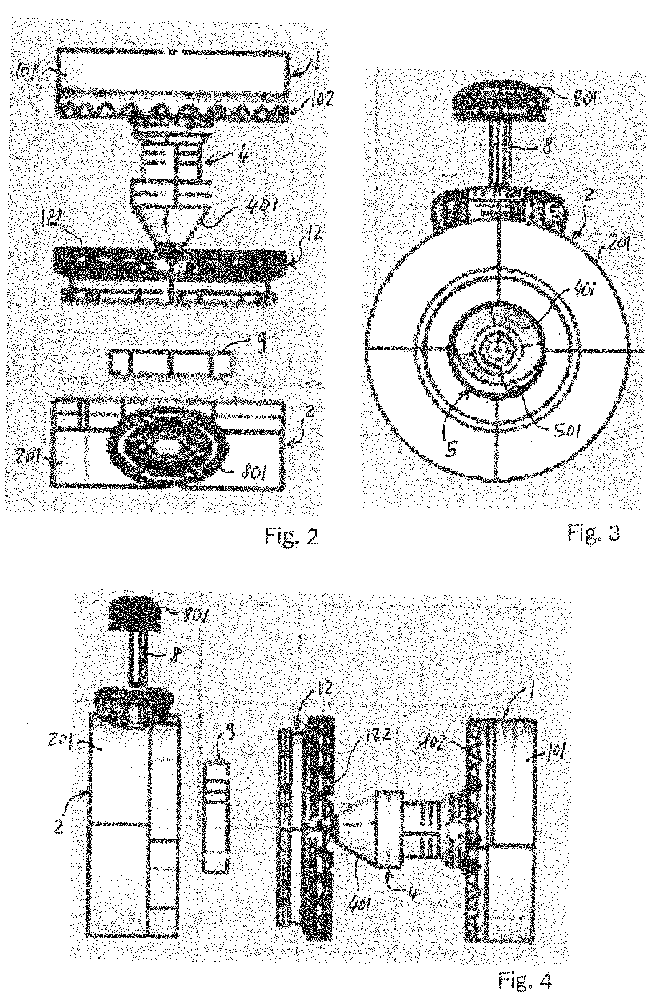

Figures 1 to 4 show different views of an embodiment of the connection module between a coupling terminal to a stump (both not illustrated since they are known) and a gripping tool or an end effector that are configured to perform certain activities and/or actions.

The module essentially comprises two parts: a first part 1 is configured to be fixed, preferably in a removable manner, to the coupling terminal of the abutment in a specific and pre-established position that can vary depending on the type of coupling terminal; a second part 2 is configured to be fixable to a gripping tool not shown in figures 1 to 4, but shown in the following figures and indicated generically with 31, 32, 41, 51, 52, 53 and 71.

In the illustrated embodiment, without this constituting a limitation, the module has rotational symmetry and the two parts 1 and 2 are respectively constituted by a cylindrical body preferably of identical external diameter and which are provided with reciprocal coupling and uncoupling members.

In the illustrated embodiment, the mutual coupling and release members consist of a coaxial central pin or hub 4 that protrudes in the direction of the second part 2, for a predetermined length and that is expected to be coaxial with the cylindrical body of the said first part 1.

The coupling pin 4 has a conical head 401 that extends towards the connection base to the cylindrical body of the part 1 with a section of stem having a diameter smaller, by a predetermined amount, than the diameter of the base of the conical head 401. Therefore, the base of the conical head 4 forms an annular shoulder for radial widening of the coupling pin 4 with respect to the stem, which annular shoulder forms a retaining surface cooperating with a radially movable retaining tooth provided in a coupling seat present in the second part 2 of the module. The conical enlarged head tapers conically towards the end axially furthest from the cylindrical body 101 of part 1 and forms a conical surface for insertion into a coupling seat 5 provided substantially coaxially with the cylindrical body 201 of the second part 2 of the connection module.

The said coupling seat 5 is constituted by a cylindrical hole for the coupling pin 4, which hole is substantially coaxial with the cylindrical body 201 of the second module part 2. The hole has a diameter substantially slightly larger than the diameter of the base of the enlarged conical head 4.

In a predetermined axial position with respect to the length of the cylindrical hole and at a predetermined distance from the frontal abutment side 6 of the second module part 2 with a facing frontal abutment side 7 of the first module part 1, at least one engagement tooth is provided which can be moved radially between two extreme positions: a position of engagement of the enlarged conical head, in which position it protrudes radially internally from said cylindrical hole of the engagement seat 5 to an extent corresponding at most to the difference in radius between the base of the conical head 4 and the connecting stem to the body 101 of the first module part 1 and a release position in which said radial tooth is moved radially outwards with respect to the axis of said hole of the engagement seat 5 to an extent such as not to penetrate inside the hole, leaving the entire passage space of the same free.

The said pre-established axial position of the radial engagement tooth is expected to be commensurate with the position of the annular shoulder defined by the base of the enlarged conical head 4 when the two parts 1 and 2 are in the end-of-stroke abutment position of mutual engagement, and such that in said position the radial engagement tooth is positioned posterior to the said annular shoulder formed by the base of the enlarged conical head 4, forming a retaining stop of the said enlarged head 4 in the direction of extraction from the engagement seat 5.

Advantageously, the said at least one radial coupling tooth is provided to be controllable in the said two end-of-travel positions of coupling and release thanks to a control actuator which is constituted by a pusher 8 which has a preferably radial actuation stroke and which is provided at its end with the said tooth or cooperates with it or alternatively which acts to activate a translation mechanism of the coupling tooth itself.

The pusher 8 is in turn operated by means of a head 801 which protrudes externally from the mantle surface of the body 201 of the second module part 2. In the illustrated embodiment, the pusher 8 is constituted by a push rod housed slidably in a radial hole 202 provided in the cylindrical body 201 of the second module part 2 and has an end external to said body 201 which connects to said head 801. The head 801 is in turn guided slidably in the axial direction of the pusher 8 thanks to a guide formed by a cup-shaped housing coaxial to said pusher and of an external shape corresponding to the perimeter mantle surface of said head 801.

According to a further preferred embodiment, the said at least one radial tooth and consequently the pusher 8 and the control head 801, are stressed by means of one or more elastic elements (not illustrated in detail) stably in the radial engagement position of the said engagement tooth, and the pusher 8 with the head 801 are dynamically coupled to the said radial tooth in such a way as to assume a radial position more external to the axis of the hole that forms the engagement seat 5, while such dynamic coupling is configured to push the said radial engagement tooth into the radial release position in which it does not protrude inside the said hole of the engagement seat 5 when, by means of pressure on the control head 801 in a radial direction towards the axis of the said hole, the said pusher 8 is moved radially inwards, i.e. in the direction of the central axis of the said hole. By releasing the pressure on the control head, the elastic elements, in turn, automatically push the group formed by the radial tooth, the pusher 8 and the control head 801 into the engaging position.

Figures 1 to 4 show a particular embodiment of the radial engagement tooth. In this case the radial engagement tooth is constituted by a sector of an internal perimeter edge 901 of a through slot, preferably circular, which is provided in a slider and which slider is in the shape of a quadrangular frame. The frame 9 is housed in a sliding guide seat 10, in a radial direction with respect to the hole that forms the coupling seat 5 in said second module part 2 and which sliding direction is substantially parallel to the radial direction of the pusher 8.

The guiding seat 10 of the frame 9 is constituted by an axial niche provided coincident with the hole 501 of the coupling seat 5 and which also has a quadrangular shape in which two opposite sides are oriented in the radial direction of movement of the quadrangular frame 9 and have a distance between them that is identical or slightly greater than the corresponding dimension of the frame 9, while at least part of the two opposite sides oriented transversely to the radial direction of sliding of the frame 9 constitute end-of-travel abutment surfaces of said radial movement of said frame 9 and have a distance between them that is greater than the distance of the corresponding sides of the frame 9 and substantially corresponding to the movement stroke.

One of the said transverse sides of the frame 9 cooperates with the pusher 8, while the opposite transverse side 902 of the frame 9 cooperates with elastic elements (not illustrated) which are interposed between the opposite transverse side 902 and the facing side 110 of the quadrangular guide seat 10.

The two transverse sides of the quadrangular seat are provided at radial distances from the edge of the cylindrical hole 501 of the coupling seat 5 so that in the condition of abutment of the transverse side of the frame 9 facing the pusher 8 against the corresponding transverse side of the quadrangular guide seat, i.e. in the position corresponding to the condition of coupling of the conical enlarged head 4 in the seat 5, the axis of the circular opening is offset with respect to the axis of the hole 501 and the offset is substantially corresponding to the radial width of the annular shoulder formed by the base of the conical enlarged head 4.

The stroke of the frame 9 in the direction of the end-of-stroke stop in which the transverse side 902 of the same abuts against the corresponding transverse side 110 of the quadrangular guide seat 10, corresponds to a movement of the circular opening of such a size as to cause the central axes of said opening and of the hole 501 of the coupling seat 5 to coincide and therefore of the circular perimeter edge 901 of said opening with the internal surface of said hole 501, freeing the passage through said opening of the enlarged, conical head 4 in the direction of extraction from said hole 501 and from the coupling seat of the second part 2 of the module.

This position of the frame 10 and of the circular aperture corresponds to a radially recessed position, by a corresponding measure, of the pusher 8 and of the control head 801, i.e. of the stroke of said pusher 8 and of said head 801 in a radially internal direction, i.e. towards the central axis of the seat 5 and/or of the body 201 of the second module part 2.

With reference to a further feature, the quadrangular guide seat 10 is made in the shape of a recess and has a bottom wall and four side walls consisting of the two side walls oriented in the sliding direction of the frame 9 and the two opposite transverse walls. On the side facing the first part 1 of the module, said quadrangular guide seat 10 is closed by an annular ring 12 which is made up of a disc that can be removably fixed to the front side of the second part 2 of the module facing the first part 1. The disc has a central circular opening, coaxial in diameter, the diameter of which is substantially identical or slightly larger than the diameter of the hole 501 of the coupling seat 5. When fixed to the body 201 of said ring, said opening is positioned axially coinciding with said hole 501 of the coupling seat 5.

The fixing can occur in any way and in the illustrated figure the said ring 12 engages with the body 201 by means of a coupling of the so-called bayonet type, the ring 12 being provided in at least two angularly spaced points with radial teeth 121 protruding from the perimeter side of the said ring, which teeth engage in undercut seats 203 provided in a cylindrical end wall 204 of engagement of the said ring 12.

Still according to a further improvement, on the side of the said ring 12 facing the first part 1 of the module the said ring 12 carries a crown of axial projections and notches indicated with 122, which notches, and which projections cooperate with a corresponding crown of axial teeth and notches provided on the facing head side of the first part 1 and which crown is indicated with 102.

In the illustrated embodiment, preferably, said tooth crowns and axial notches are in the form of a surface with a rounded shape, sinusoidal or similar, which have an identical shape to each other. These crowns 122 and 102 allow said two parts 1, 2 of the modules to be locked together in a relative angular position chosen by the user and which can be modified at each coupling of the two parts of the module 1, 2 one to the other.

As will appear more clearly from the following description, the aforementioned construction allows the control head 801 and therefore the pusher 8 to be operated in such a way as to perform the release stroke according to the methods described above, generating the pressure force on the control head by means of a shaped cam surface for operating the control head 801 and the pusher 8.

According to the most general embodiment, the actuation member of the combination of control head 801, pusher 8 and radial tooth, or coupling frame 9, is made up of two opposing wedging walls of said second part 2 of the connection module in which said part 2 is inserted in such a position that in the wedging end-of-stroke condition the sliding axis of the control head 801 and of the pusher 8 as well as of the frame is oriented substantially in the direction of the distance between said two opposing wedging walls. These form an insertion channel for part 2 of the module with an entry opening at which the distance between said two opposing walls is greater than the maximum dimensions of the second part of module 2 between two opposing sides of said body 201 and in particular the diametral dimensions of the body 201 of said second part 2 and in addition the radial protrusion of the control head 801 in its condition of maximum protrusion beyond the perimeter wall of the casing of said body 201.

Said two opposing walls converge with each other to such an extent that, in the final wedging position of said second part 2 of the module between them, the distance between said two opposing walls substantially corresponds to the diametrical dimension of said body 201 plus the radial protrusion of the control head 801 in the radially recessed position towards the axis of the hole 501 of the coupling seat 5.

The shape of the two walls can be substantially rectilinear so as to determine a wedging channel of the said second part 2 along which the said part 2 is moved according to a rectilinear trajectory from the entry end to the end of the wedging stroke, bringing the said second part 2, with the sliding axis of the pusher 8 and the head 801 in an angular position, in which the said sliding axis is oriented substantially transversely to the said rectilinear wedging path, at least for a terminal part of the said wedging path.

Alternatively, the shape of the opposing wedging walls can be at least partially curved, at least for a final section of the wedging stroke, so that the wedging path provides an initial rectilinear stroke and a final rotational stroke around the axis of the coupling seat 5 and/or of the coupling pin 4.

It should be noted that although in the present description, the coupling seat is provided in the second part 2 of the connection module which is fixed or fixable to a gripping tool or to another kind of end effector, it is also possible to provide an embodiment in which, "mutatis mutandis", the coupling seat is associated with the first part 1 of the module and the coupling pin with said second part of the module.

With reference to an embodiment, said two opposing walls are formed by two lateral walls of a U-shaped band of a wall whose bottom side constitutes a limit stop of the wedging stroke or a rotational guide surface of the body of said second part 2.

The following figures show various possible alternative embodiments which constitute examples of application of the general configurations described above.

In the examples shown, the cam-shaped surfaces are each integrated with a parking cradle for a gripping tool and/or an end effector, with different gripping tools or different end effectors intended for carrying out different activities being shown in the figures and all provided with an identical second part 2 of a connection module that can be coupled to the said first part 1 of the module fixed to the coupling terminal of the stump.

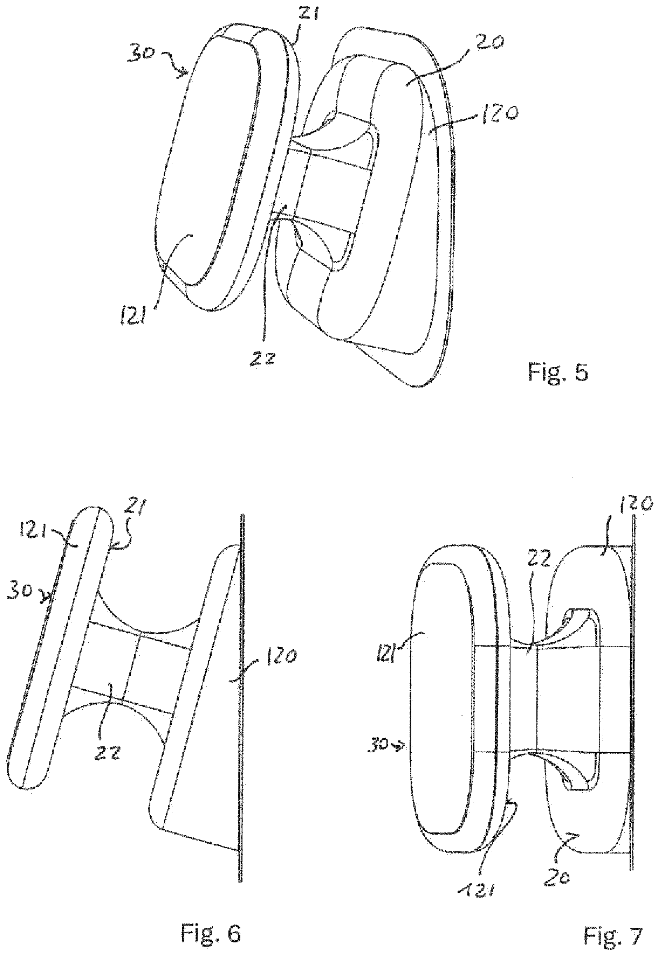

Figures 5 to 7 show a parking cradle 30 for a maximum of two different gripping tools or end effectors. In this case, the two opposing wedging walls indicated by 20 and 21 are made up of the facing internal walls of a base plate 120 and of an opposite front plate 121 which are spaced apart from each other to a lesser extent than the maximum diametral dimension of the second module part 2 along a diametral axis coinciding with the sliding axis of the pusher 8 and the associated control head 801. The convergent section of the said two walls 20 and 21 is created by the rounded edge of the perimeter sides of the said plates 120 and 121, while the said two plates are held at the said distance by a central connecting element 22.

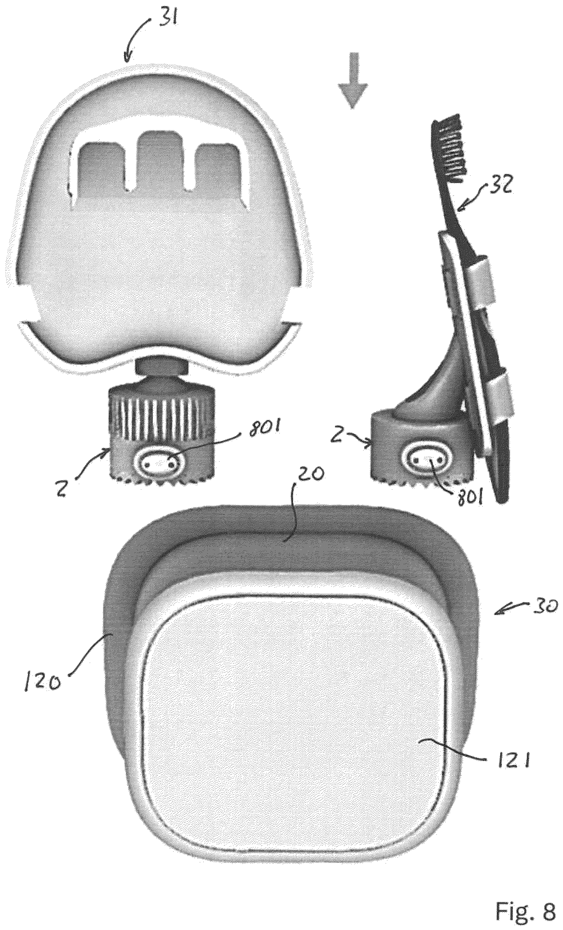

Figures 8 to 12 show the parking cradle 30 in combination with two different gripping tools or end effectors indicated with 31, 32 which have, at one fixing end to the socket for coupling to the stump (not illustrated), a base formed by the aforementioned second module part 2 which is made according to any of the embodiments described above and which bases are identical to each other.

One of the two end effectors 31 is made in the form of a gripping device or hand while the other of the two end effectors 32 is constituted by a support for the movable fixing of a body care tool, such as a toothbrush or similar and has a cradle for fixing the handle of said toothbrush provided in combination with transverse tightening elements, such as bands, straps, clips, or similar.

In figure 8, the two end effectors 31, 32 are illustrated in the position immediately preceding insertion into the corresponding parking and wedging cradles between the walls 21 and 22 of the same. The insertion takes place following a straight path in the vertical direction of at least the base formed by said part 2 of the connection module which can be made according to any of the possible execution variants described above.

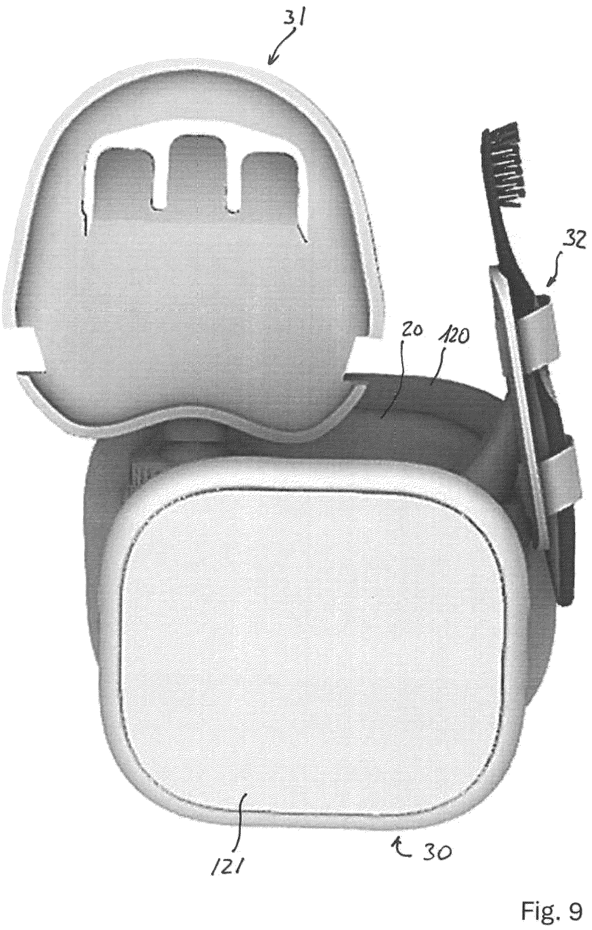

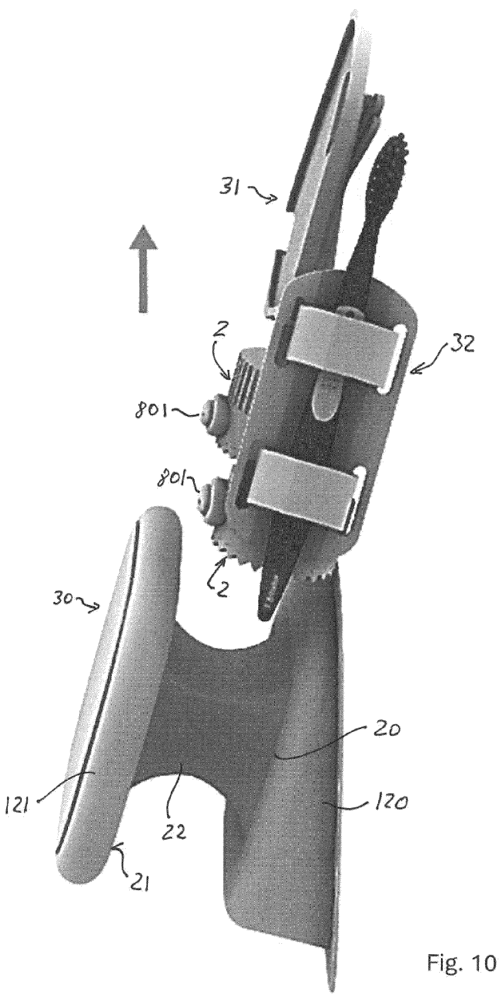

Figure 9 shows a front view of the parking cradle 30 in which the two end effectors 31 and 32 are inserted in the final parking position. Figure 10 is a lateral view on one side of said parking cradle 30 intended to house the end effector 32 and in the position substantially corresponding to that of figure 9.

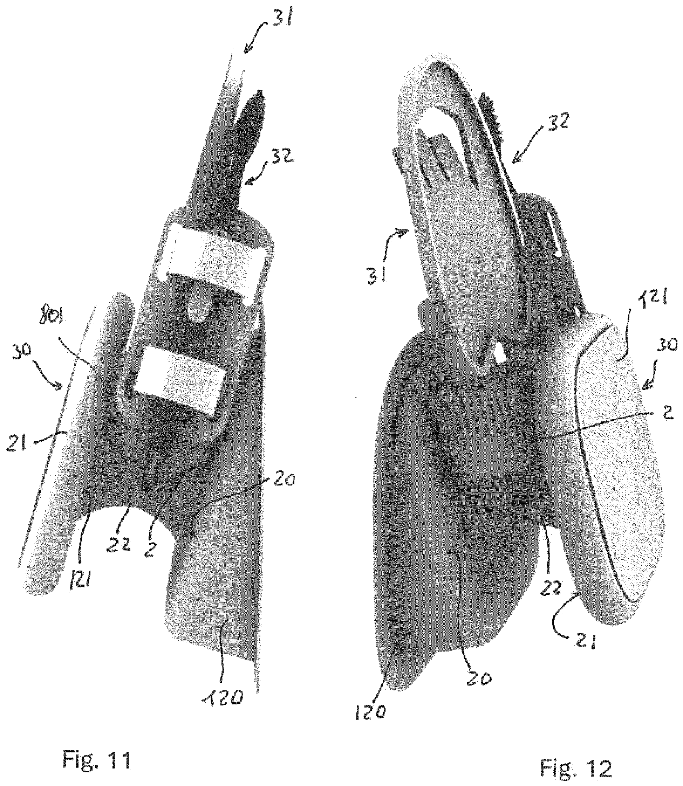

The views of figures 11 to 12 are side views of the parking cradle 30 with the respective end effectors 31, 32 in the final parking position in said parking cradle 30 and in which the wedging condition of the second part 2 of the connection module between said two opposing wedging walls 20, 21 is visible, in which parking position the control head 801 is pushed radially inwards towards the coupling seat 5 for the coupling pin 4 for coupling the second part 2 to said first part 1 of said connection module which is not visible in the figure but shown in the previous figures 1 to 4 and thanks to which, the radial coupling tooth which in the present example is formed by a sector of the perimeter edge 901 of the opening provided in the sliding frame 9, is placed in the release position of the coupling pin 4, or of non-interference with the annular shoulder of the conical enlarged head 401 of the coupling pin 4 provided on the said first part.

In this condition, therefore, the user is able to disengage the first part 1 of the connection module from the second part 2 of the same and therefore to free the coupling terminal to the stump on which the said first part 1 of the module is fixed from the said second part 2 of the module and from the corresponding end effector 31, 32 which remained retained in the parking cradle 30.

By performing reverse movements, it is possible to couple the first part of the module 1 to the second part of the module 2, performing a translation movement with the coupling terminal to the stump to insert the coupling pin 4 into the coupling seat 5 and automatically hook the said pin to the said seat by means of a further extraction stroke, substantially in the same direction as the said end effector 31, 32 from the parking cradle 30, therefore moving from the position shown in figures 9 and 11 and 12 to that of figures 8 and 10. All this does not require any action on the part exercised by the twin limb and/or by a third person.

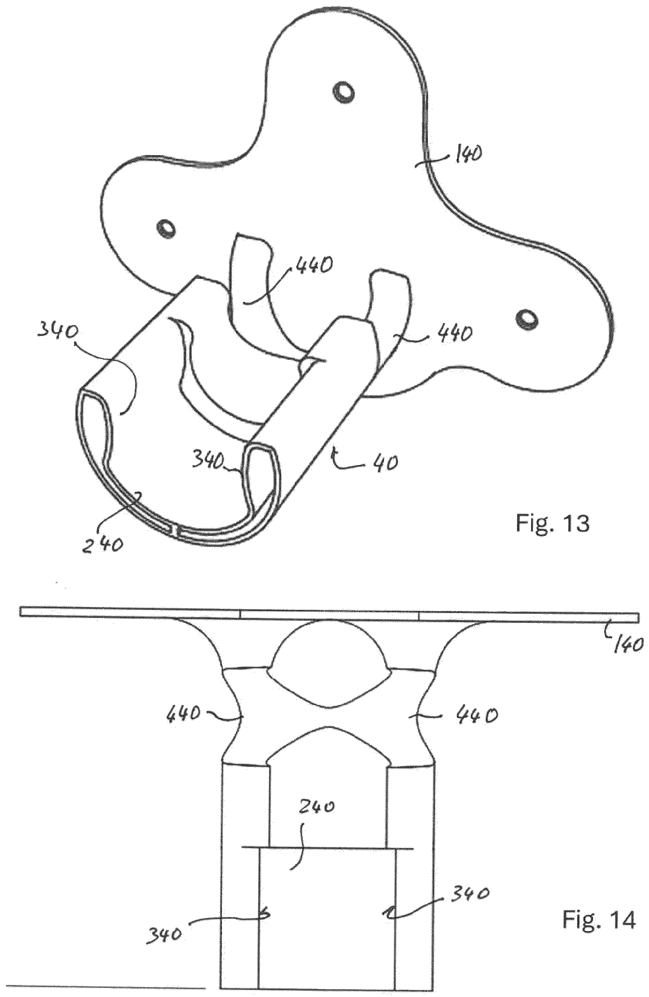

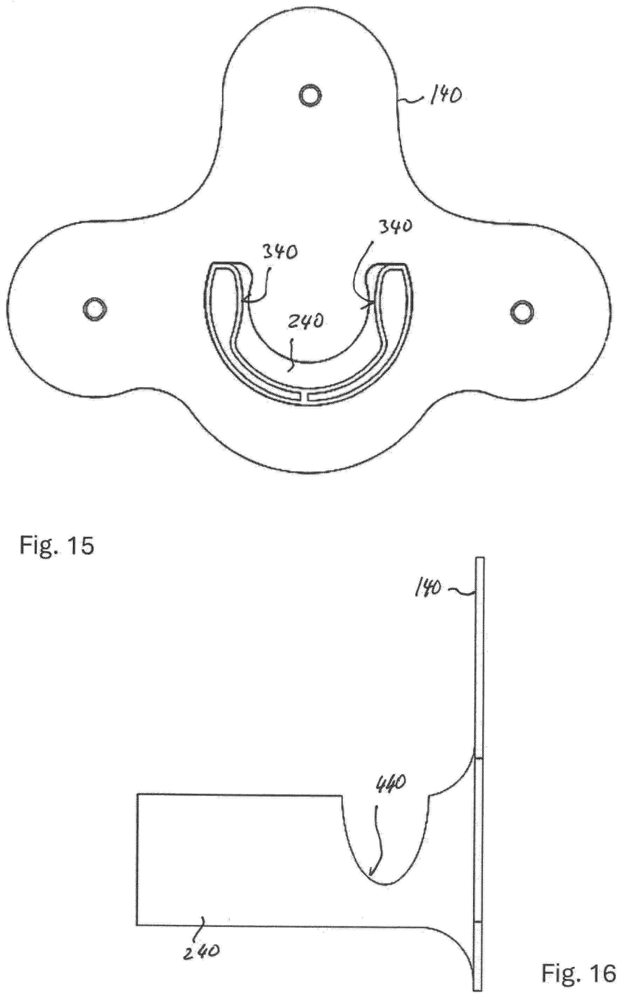

Still remaining in the field of end effectors or gripping tools for personal intimate care, figures 13 to 20 show a further embodiment in which the end effector indicated with 41 is made in the form of a shower terminal.

In this case, the parking cradle indicated with 40 has a wall fixing base 140 from which a surface with a channel cross-section 240 departs in a direction perpendicular to said base and which is curved according to a trend substantially coaxial to the circular shape of the body 201 of the second part 2 of the module, while the two opposing branches along the edges that delimit the opening of said channel surface 240 have convex radial recesses 340 that constitute the opposing converging wedging walls, said wedging being performed by means of a rotary path of said second part 2 and of the relative end effector 41 inside said channel surface 240 and substantially coaxially to the same.

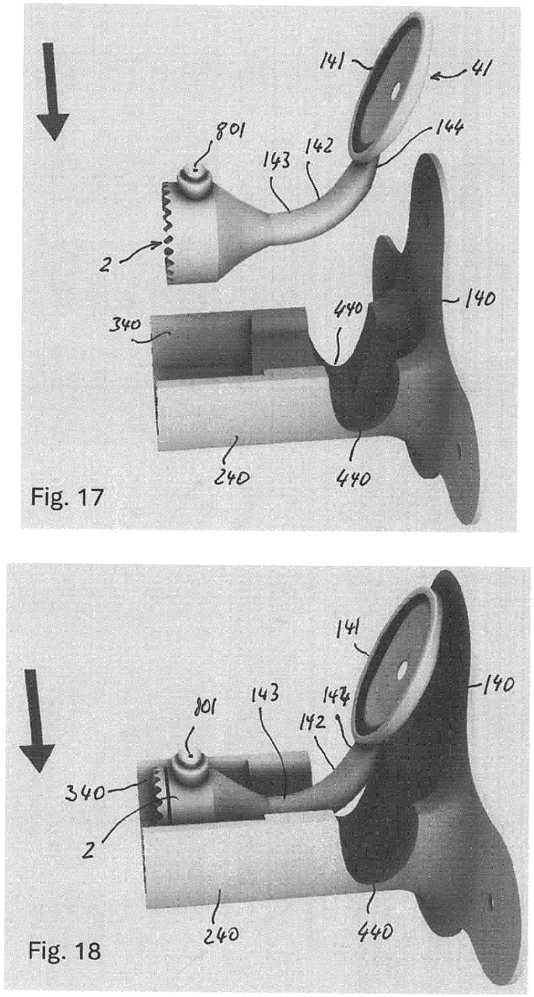

The end effector 41 shown in figures 17 to 20 has a sort of irrigation head 141 that is connected by means of a curved stem 142 with a base section 143 substantially coaxial to said second part 2, while the parking cradle has in the two opposite side walls of the channel surface 240 and in a diametrically coinciding position with each other a slot 440 for housing the angled terminal branch 144 of said end effector and which allows said end effector and therefore also module part 2 to be rotated by substantially 90°.

Figures 17 to 20 show the insertion path of the end effector 41 into the parking cradle 40 and the wedging path of module part 2.

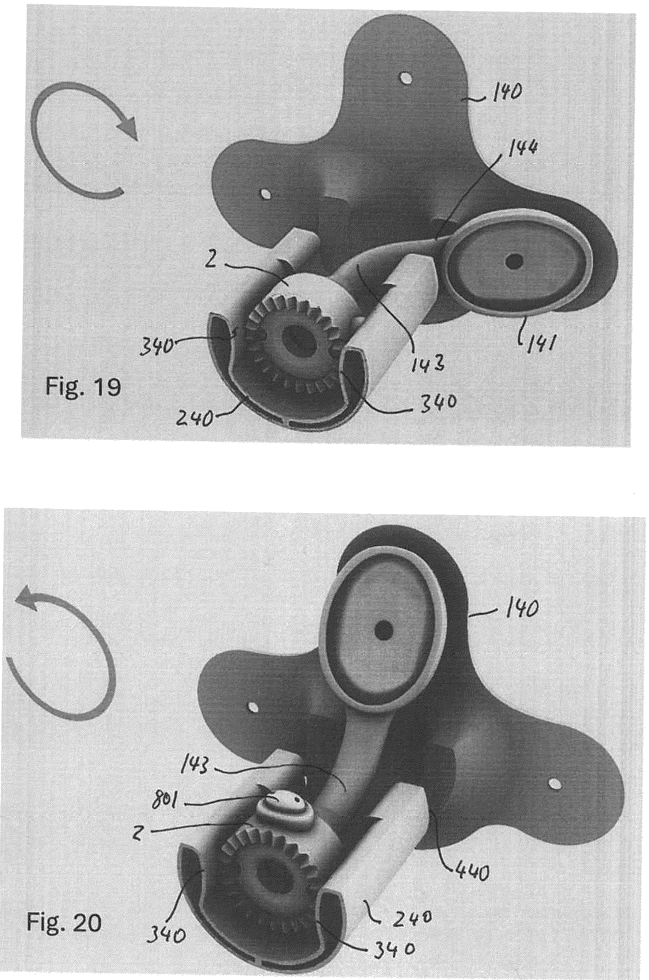

With reference to figure 17, the end effector is inserted into the parking cradle 40, i.e. into the channel surface 240 with its angled branch 143 oriented vertically and with the control head 801 of the pusher 8 also oriented upwards. With a vertical downward movement, the end effector 41 is brought inside the said channel surface 240. Figure 19 shows the final wedging position of the second module part between the said opposing convex protrusions 340 in which one of the said two convex protrusions 340 cooperates with the control head 801 of the pusher 8 by pushing it in a radial direction towards the seat 5. This wedging occurs by means of a rotation of substantially 90° starting from the position of figure 18 and as indicated by the arrow.

As shown in figure 20, it is also possible to perform a wedging rotation in the opposite direction to that of figure 19.

Similarly, to the previous example, also in this embodiment, the end effector 41 is engaged with module part 2 to module part 1 fixed to the coupling terminal to the stump (not illustrated). The release of the end effector 41 in the parking cradle 40 and the automatic release of the first module part 1 from the second connection module part 2 takes place by means of simple insertion by means of vertical translation and subsequent actuation of the control head and the pusher in the release position of the pin 4 from the seat 5 by wedging the said second part 2 and the said control head 801 between the said two convex recesses 340. In this way, the part 1 of the connection module is automatically released from the second part 2 of the said module and therefore the coupling terminal to the stump is released from the end effector and can be coupled to a further different end effector.

The removal of the end effector 41 from the parking cradle 40 with the simultaneous automatic coupling of the first part 1 to the second part 2 of the module is obtained thanks to the execution of the reverse sequence of the steps described above.

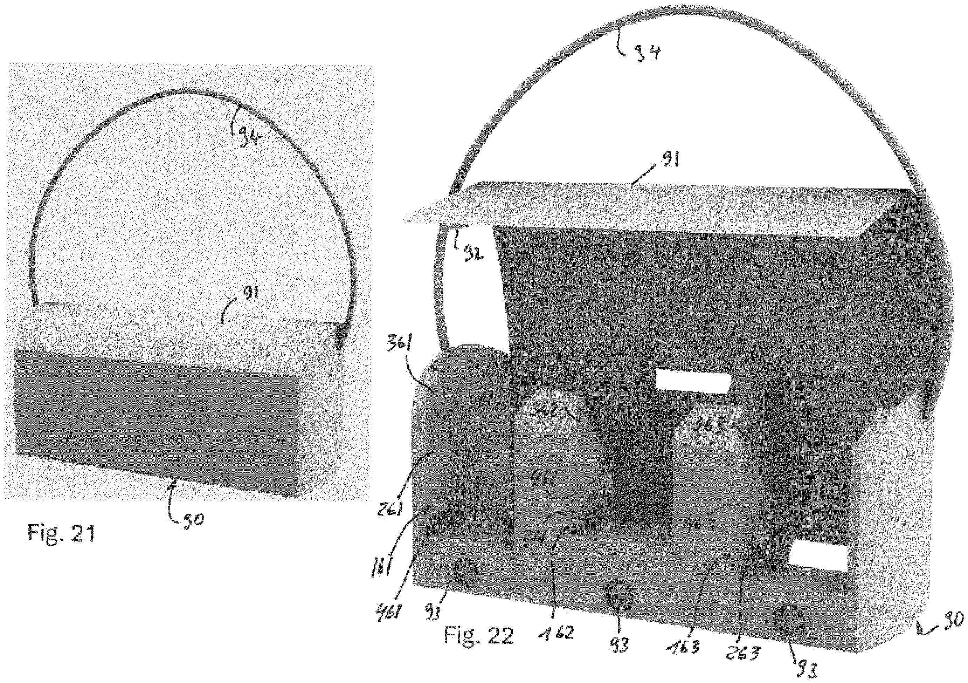

Figures 21 to 25 illustrate a third embodiment of the invention. In this embodiment, three different gripping tools are provided, indicated respectively by 51, 52, 53, which gripping tools are each housed in a dedicated parking cradle of a transportable parking warehouse made in the shape of a box.

Figures 21 and 22 show said transportable box in the closed condition and in the open condition with the three parking cradles 61, 62, 63 accessible to the view and insertion of the corresponding gripping tool 51, 52, 53.

In particular, the transportable box indicated globally with 90 comprises a swinging upper lid 91 which is articulated to a perimeter edge of said box, in particular the upper edge of the rear side of said box 90. The lid 91 and the box 90 have cooperating mutual fastening members indicated with 92 and 93 and which, in the illustrated embodiment, are provided on a front terminal band of the lid 91 intended to overlap the front side of the box 90 when the lid is in the fully closed position. A handle 94 allows the box to be gripped for transport. In particular, in the illustrated embodiment, said handle is in the form of an arched element which extends from one to the other of the two opposite side walls of the box 90 which are oriented transversely to the axis of oscillation of the lid and the shape of said handle 94 is such as to allow the lid 91 to pass under it for the complete opening of the box 90.

Figure 21 shows the box in the fully closed condition with said closing members 92, 93 coupled together in a removable manner. Figure 22 shows the lid in an intermediate position of oscillation in the direction of opening of the box 90 and of partial opening of the same. Each parking cradle has a housing compartment for the associated end effector, and which is shaped in a manner corresponding to the external shape of said end effector and each parking cradle is open at the top and in correspondence with a front side.

In the direction of the said front side, the housing compartment extends with a channel section indicated respectively with 161, 162 and 163 and which channel section has a bottom wall and two opposing side walls 261, 262, 263, which side walls are shaped so as to have an initial upper section 361, 362, 363 converging towards that of the opposing side wall and which extends respectively with a lower, terminal section 461, 462, 463, having a substantially constant distance from the corresponding lower, terminal section of the opposing side wall, thus forming a progressive narrowing of the wedging of the second part 2 of the module fixed as a base to the corresponding gripping tool 51, 52, 53.

In the illustrated embodiment, the initial upper sections of the 361, 362, 363 of the opposing side walls 261, 262, 263 are made curved in a substantially convex way since, as will be seen below, the insertion path of the gripping tools 51, 52, 53 into the respective parking cradle 61, 62, 63 is substantially similar to that of the example according to figures 13 to 20.

Furthermore, as it appears evident, the said channel extension section of the parking cradles is provided in such a position that, with the gripping tool correctly aligned with the housing compartment of the corresponding cradle, the said extension section is coincident and aligned with the part 2 of the module to which the corresponding gripping tool is fixed.

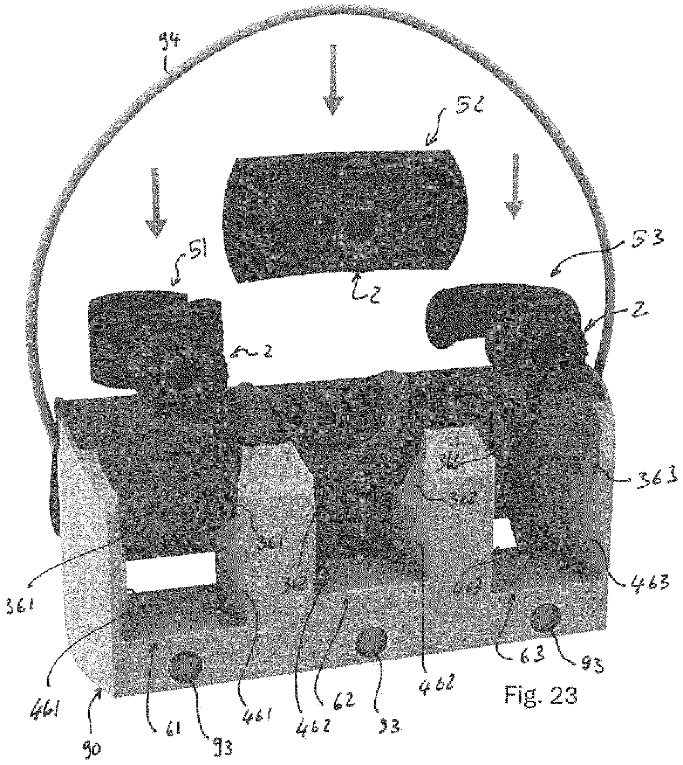

In this way, as shown in figure 23, the individual gripping tools 51, 52, 53 with the respective second part 2 of the connection module are brought into a position vertically aligned respectively with the part of the tool with the housing compartment of the corresponding parking cradle 61, 62, 63 and at the same time with the corresponding second part 2 of the connection module in a position vertically aligned with the channel extension 161, 162, 163.

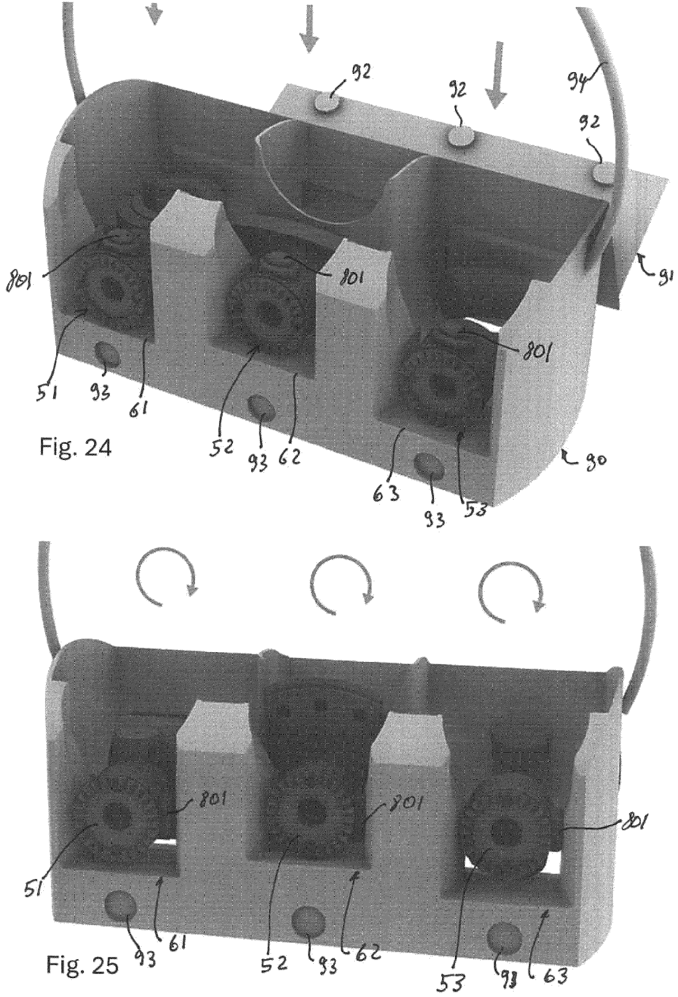

The insertion of the gripping tools 51, 52, 53 into the parking cradles 61, 62, 63 occurs as shown by the arrows by means of a translation in a vertical direction, downwards, until reaching the position shown in figure 24. In this insertion phase, the gripping tools are oriented in such a way that the control head 801 of the pusher 8 is facing upwards and therefore the coupling condition of the first part 1 of the connection module fixed to the coupling terminal to the stump with the second part 2 of said connection module fixed to a gripping tool 51, 52, 53 is maintained.

According to the present embodiment, to operate the coupling and release members so that they assume the release condition of the first part 1 from said second part 2, one of the gripping tools 51, 52, 53 of the gripping tool group with its corresponding second part 2 of the connection module is rotated around the axis of the coupling pin 4 and of the coupling seat 5, causing the control head 801 of the pusher 8 to cooperate with the lower terminal part 461, 462, 463 of the corresponding cradle 61, 62, 63 and wedging said second part 2 of the connection module between said two terminal parts 461, 462, 463 of the two opposing lateral walls 261, 262, 263 of the channel extension 161, 162, 163, as shown in figure 25 and by the arrows indicating the wedging rotation.

To automatically attach and remove from the box the second part 2 of the connection module to which a corresponding gripping tool 51, 52, 53 is fixed to the first part 1 of the connection module secured on its turn to the coupling terminal to the stump, it is sufficient to carry out the movements described above in reverse sequence. These movements involve inserting the coupling pin 4 of the first part 1 into the coupling seat 5 of the second part 2, bringing the two parts 1 and 2 into mutual contact with the two axial tooth crowns, and then rotating the first and second parts 1, 2 together in the opposite direction to the wedging direction shown by the arrows in figure 25. In this way, the control head is no longer radially stressed inwards, and, thanks to the action of the elastic elements, the coupling organs are activated, bringing the sliding frame 9 and the perimeter edge 901 delimiting the opening provided in the same into the position of axial overlap with the base of the conical enlarged head 4.

According to a variant embodiment, it is also possible to obtain the automatic coupling of the first part 1 to the second part 2 even just by only vertically translating the gripping tool upwards and therefore freeing the corresponding second part 2 of the connection module from the wedging position.

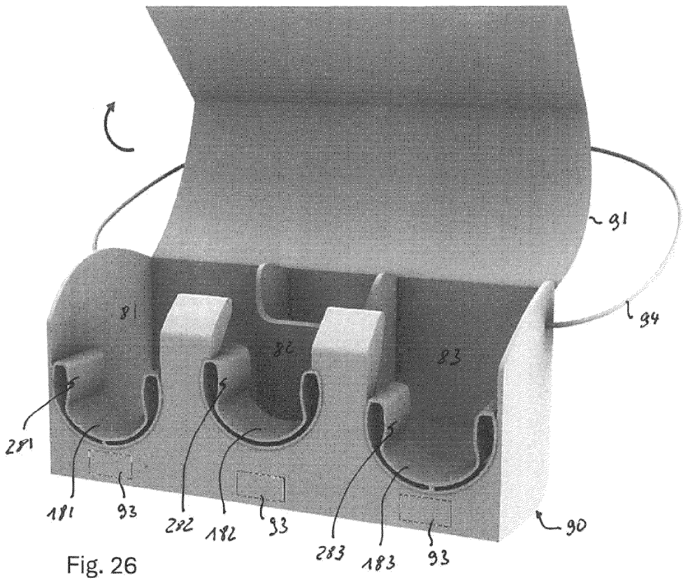

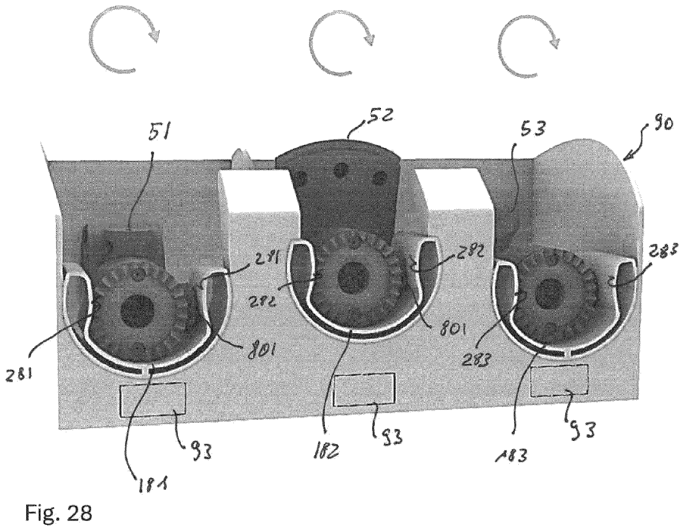

Figures 26 to 28 show, similarly to the previous figures, an variant of the embodiment which provides a transportable box 90 with at least one, two or more and in particular three parking cradles for the same number of gripping tools which are indicated with 51, 52, 53. In figures 26 and 28, the same reference numbers used in figures 21 to 25 will be used for identical parts.

As shown in figures 26 to 28, this variant differs from the embodiment according to figures 21 to 25 essentially in that the parking cradles are made in a substantially identical way to those according to the embodiment of figures 13 to 20.

In particular, each parking cradle 81, 82, 83 is made from a channel element 181, 182, 183 which is open at the top and whose side walls have convex radial recesses 281, 282, 283 which progressively reduce the width of the opening of the section of said channel element forming opposite converging wedging walls. The said channel element 181, 182, 183 extends axially for at least a band of predetermined depth which starts from the front wall of the box 90, and whose depth is substantially corresponding to the axial extension of the cylindrical body 201 of the second part 2 of the coupling module which is integral with the gripping tool 51, 52, 53, in such a way that, similarly to the embodiments of figures 13 to 20 but also of figures 21 to 25, the cylindrical body 201 of the corresponding gripping tool 51, 52 and 53 can be inserted vertically inside the channel element 181, 182, 183 of the corresponding parking cradle 81, 82, 83 thanks to a vertical translation towards the inside of the said channel element and in an angular position of the said cylindrical body 201, in which the control head 801 of the pusher 8 is facing upwards, while a thrust is exerted on the said control head 810 in the direction of translating the pusher 8 in the direction in which the coupling seat is in the inactive position, i.e. the release of the pin 4, thanks to the rotation of the gripping tool 51, 52, 53 around the axis of the cylindrical body 201 of the said second part 2 through an angle of substantially 90° and with which the said control head 801 is brought to cooperate with the apex of one of the convex radial recesses 281, 282, 283 of the lateral walls of the corresponding channel element 181, 182, 183.

Advantageously as also in the embodiment of figures 13 to 20, the cross-section of the channel elements 181, 182, 183 is empty and is delimited by a mantle wall having a certain predetermined thickness which, in combination with the characteristics of elastic deformability of the material that constitutes said mantle walls, a certain elastic compression action of the control head 810 is also guaranteed.

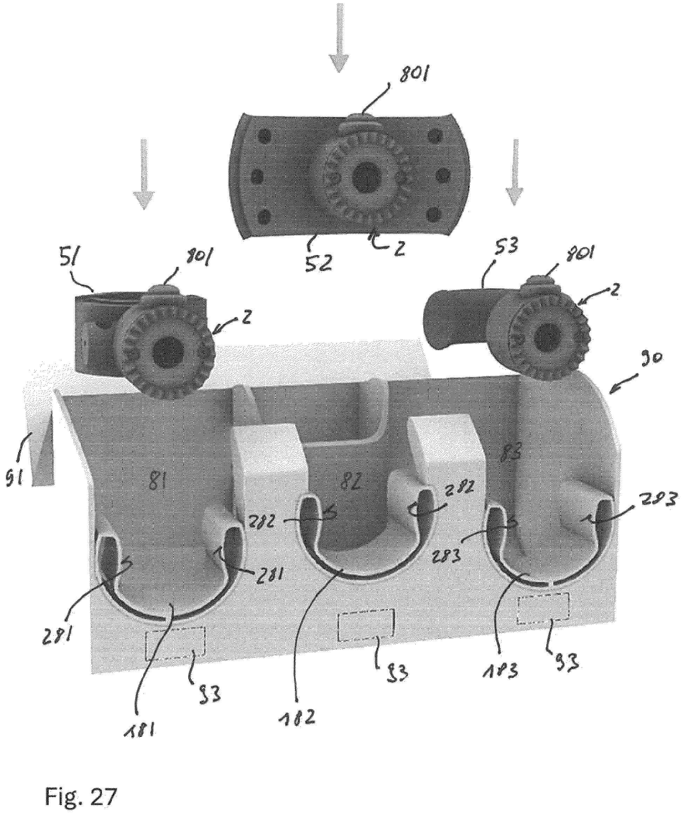

Figures 27 and 28 respectively show the insertion path of the gripping tools 51, 52, 53 in the respective cradles 81, 82, 83 by means of a vertical insertion translation with the control head 801 facing upwards and the position in which said gripping tools are rotated coaxially to the cylindrical body 201 of the respective part 2 by an angle of 90° in the position in which the cylindrical body 201 and the control head 801 are wedged between the convex radial recesses 281, 282, 283 of the corresponding cradle 81, 82, 83 and in which the control head is pressed radially inwards towards the central axis of the cylindrical body bringing the coupling seat into the inactive coupling or release condition.

As indicated for the embodiment of figures 21 to 25, the automatic collection and coupling of one of the gripping tools on the part 1 fixed to a coupling terminal to a stump is obtained by reversing the deposit and release sequence referred to in the previous description referring to figures 27 and 28.

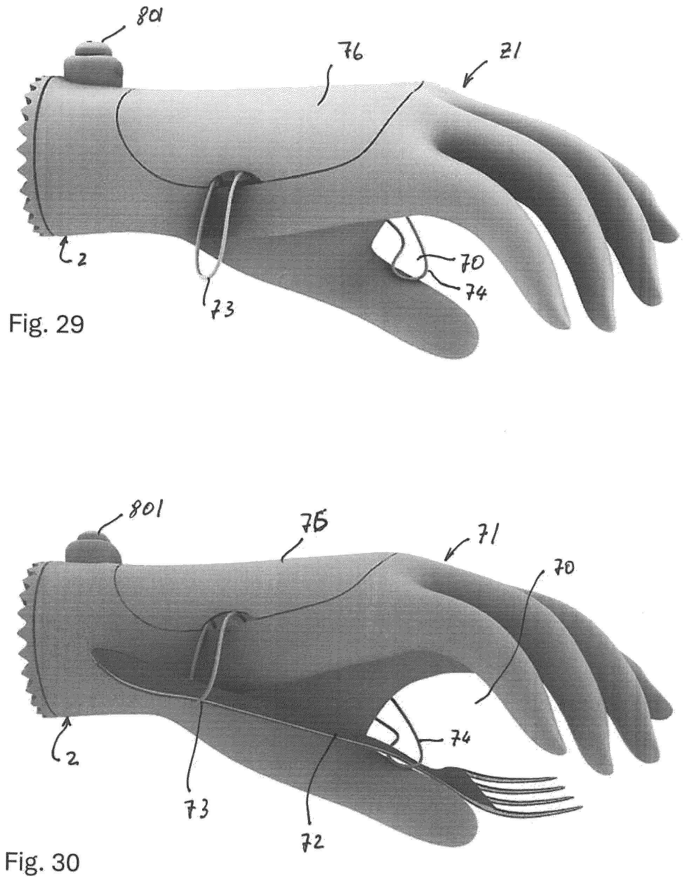

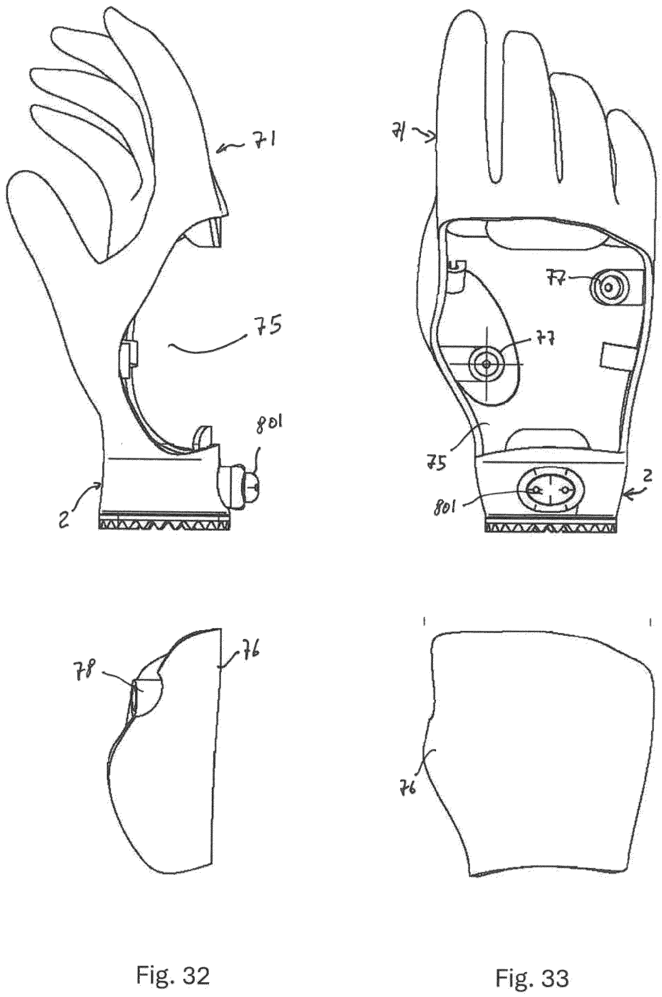

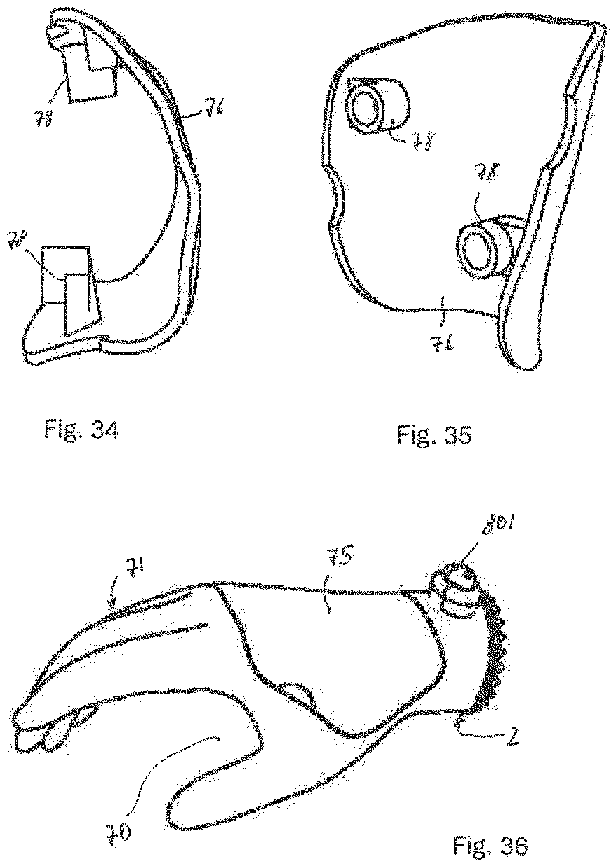

Figures 29 to 36 show different views of a particular embodiment of a gripping tool that can be envisaged in combination with the features of the present invention according to one or more of the embodiments and variants described above.

This embodiment of the gripping tool is particularly suitable for gripping and handling tools that have a handle with a longitudinal extension that is dimensionally preponderant with respect to the transverse extension. This is for example the case of cutlery or other similar utensils.

Figure 30 shows the gripping tool 71 in combination with a fork 72.

The gripping tool 71 is shaped like a hand having a fixed position in which the thumb is opposite the remaining fingers so as to form a relatively large housing channel for the tool 70 and with an entry port which is also relatively large and such as to allow the introduction of handles having a relatively large cross-section, such as knife handles, hammer handles or other tools.

The tool 70 is held in position at the two opposite sides of the palm of the hand-shaped element by means of an elastic band 73, 74 which forms a loop into which the corresponding part of the handle of the tool, in this case the fork, is inserted.

The elastic band 73, 74 may already be tensioned in such a condition that, upon insertion of the handle of the tool 70, it exerts an elastic clamping action of the handle of said tool against the palm of the hand element.

Alternatively, the elastic band may be provided in combination with tensioning and slackening devices, for example devices for lengthening and shortening the segment that forms the loop.

Devices of this type may be made in any way and may for example consist of tensioning/slackening mechanisms of the type produced by Boa Technologies Inc. or by other manufacturers and described by way of non-limiting example in document WO2018160583A1.

The tensioning and loosening devices can also be of a motorized type, for example by providing a winding core of at least one end of an elastic band that is driven by rotation by an electric motor and which can be controlled by buttons to operate rotation in one direction or the opposite direction, with additional locking and unlocking devices for said core relative to its free rotation.

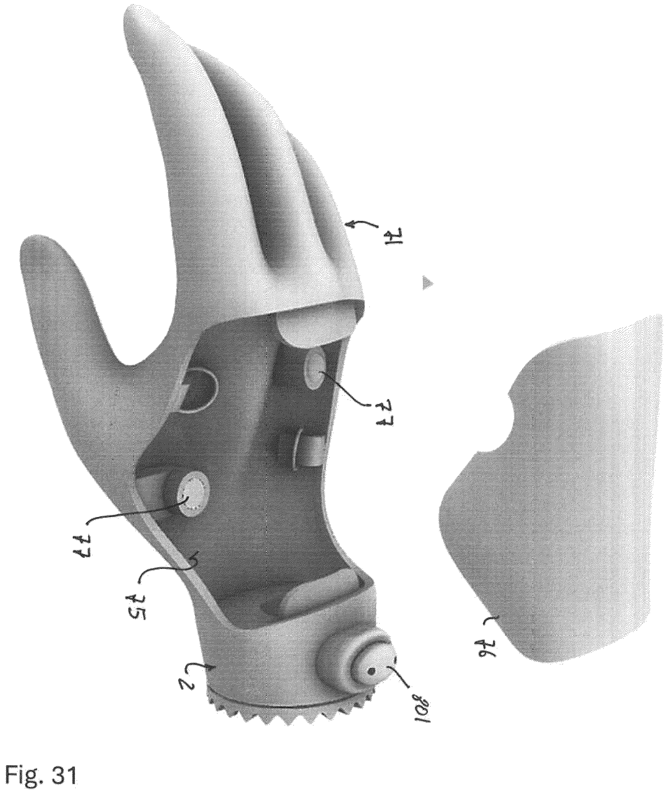

As is evident from figures 31 to 36, the hand is made hollow at least in the part relating to the back of the hand and said cavity 75 can be closed by means of a cover 76.

The cover 76 is shaped so as to complete the shape of the back of the hand. The cavity 75 can be used to house the tensioning and loosening mechanisms of one of both elastic bands and any one or more motors, as well as the power supply and electrical and control circuits of the same.

As is evident from the figures, in particular from figures 31 and 33 to 35, the fixing of the lid 76 can take place thanks to interlocking elements 77 present in the cavity 75 of the hand and which cooperate by means of shape coupling and/or by force with corresponding interlocking seats 78 provided on the lid 76.

Alternatively, it is possible that the retention in position of the lid on the hand is obtained exclusively by means of magnetic elements which are provided in coinciding positions in the cavity 75 of the hand and on the lid 76.

Such magnetic elements can also be provided in combination with the interlocking elements to strengthen the mutual coupling between the hand part and the lid and/or to generate a guiding force in the correct relative position of said interlocking elements.

It is also possible to provide alternatively or in combination different reciprocal removable fixing elements, of the cover to the hand.

As regards the elastic bands, the terminal ends of the same are guided and provided inside the cavity 75 of the hand inside which said ends can be reached to free them from fixing means and provide for the replacement of the corresponding elastic band with a new elastic band.

The hand part ends at the distal end from the fingers with a coupling module part 2 which is made corresponding to any of the embodiments described above and thanks to which to the specific advantages relating to the configuration of said gripping tool adds to the advantages obtained thanks to the configuration of said coupling module as described above.

## Claims

1. Connection module of a gripping tool, so-called end effector, to a stump coupling terminal, which connection module has a first module part (1) intended to be fixed to said stump coupling terminal and a second module part (2) intended to be fixed or to be connected integrally to a gripping tool (31, 32; 41; 51, 52, 53; 71), while said first part (1) and said second part (2) are provided with mutual coupling and uncoupling members (4, 5),
and in which the said first and/or the said second part (1, 2) have an actuator (8, 801) for controlling the said mutual coupling and uncoupling members (4, 5) alternatively from one to the other of the said mutual coupling and release conditions;
**characterized in that**
said control actuator (8, 801) cooperates with an actuation member of said control actuator to activate the mutual coupling and uncoupling members (4, 5) of said two parts (1, 2) of the connection module;
and in which the said actuation member (8, 801) is provided in a pre-established position in a parking cradle (30; 40; 61, 62, 63; 81, 82, 83) of the said gripping tool and/or of the said end effector (31, 32; 41; 51, 52, 53; 71) and cooperates with the said actuator (8, 801) during an insertion stroke of the said tool and/or of the said end effector (31, 32; 41; 51, 52, 53; 71) in the said cradle (30; 40; 61, 62, 63; 81, 82, 83) and/or during a withdrawal stroke of the said tool and/or of the said end effector (31, 32; 41; 51, 52, 53; 71) from the said cradle (30; 40; 61, 62, 63; 81, 82, 83), automatically determining the activation of the mutual coupling and uncoupling members (4, 5) respectively:
in the condition of mutual uncoupling of the said first from the said second part (1, 2) of the connection module, when with the movement of the stump the said module with the corresponding gripping tool and/or with the corresponding end effector (31, 32; 41; 51, 52, 53; 71) are brought into a parking condition inside the said parking cradle (30; 40; 61, 62, 63; 81, 82, 83) and thus freeing the first part (1) of the module from the second part (2) of the module, and
in the condition of mutual coupling of the said first part (1) of the connection module to the said second part (2), when with the movement of the stump the said first module part (1) is brought into a position ready for coupling to the said second part (2) of the said module which is provided as a base of a pre-established gripping tool or a pre-established end effector (31, 32; 41; 51, 52, 53; 71) and subsequently said first module part (1) and said second module part (2), with the corresponding gripping tool and/or with the corresponding end effector (31, 32; 41; 51, 52, 53; 71), are extracted along a pre-established path from the said parking condition inside said parking slot, extracting it from said parking cradle (30; 40; 61, 62, 63; 81, 82, 83) and thus making said connection module usable with the corresponding gripping tool or with the corresponding end effector (31, 32; 41; 51, 52, 53; 71).

2. Module according to claim 1, wherein the control actuator is constituted by a pusher (8) provided with a sliding head (801) at its end and with which said pusher (8) protrudes externally from a perimeter face of the mantle of said second part (2) of the module, while
said pusher (8) is supported to slide in both directions in a direction transverse to said perimeter face and
while the said actuating members are constituted by a cam shaped surface having a pre-established position with respect to the parking position of the gripping tool and/or the end effector (31, 32; 41; 51, 52, 53; 71) in the said parking cradle (30; 40; 61, 62, 63; 81, 82, 83)
and with a shape of the surface of said cam so that upon insertion of the gripping tool and/or of the end effector (31, 32; 41; 51, 52, 53; 71) into said parking cradle (30; 40; 61, 62, 63; 81, 82, 83) and upon extraction of the gripping tool and/or of the end effector (31, 32; 41; 51, 52, 53; 71), the said sliding head (801) slides along the said cam shaped surface, controlling the actuator of the coupling and uncoupling members (4, 5), in the corresponding condition of coupling of the said first part (1) to the said second part (2) of the connection module or alternatively in the corresponding condition of uncoupling of the said first part (1) from the said second part (2) of the said connection module respectively with the execution of the insertion path of the said gripping tool and/or of the said end effector (31, 32; 41; 51, 52, 53; 71) in the said parking cradle (30; 40; 61, 62, 63; 81, 82, 83) and with the execution of the path for extracting said gripping tool and/or said end effector (31, 32; 41; 51, 52, 53; 71) from said parking cradle (30; 40; 61, 62, 63; 81, 82, 83).

3. Module according to claims 1 or 2, in which the said pusher (8) is stably stressed by one or more elastic elements in a position of end stroke having a greater protrusion outward from the said face of the outer mantle wall of the said second and/or of the said first part (1, 2) of the said connection module and the shaped surface of the cam has a shape such that in the terminal parking position of the gripping tool and/or of the end effector (31, 32; 41; 51, 52, 53; 71) in the said parking cradle (30; 40; 61, 62, 63; 81, 82, 83), the said pusher (8) is moved against the action of the said elastic element(s) in an end position of minor protrusion outward from the said face of the said perimetral mantle wall and while during the extraction path of the gripping tool and/or of the end effector (31, 32; 41; 51, 52, 53; 71) the shape of the said cam surface is such that the said pusher (8) is progressively released and returns to the aforementioned end-of-stroke position, of maximum protrusion outwards, from the said perimetral mantle wall.

4. Module according to one or more of the previous claims, in which the mutual coupling and uncoupling members of said first part (1) and said second part (2) of the connection module are respectively constituted by a central coupling pin (4) with an enlarged head (401) protruding from one side of the head of the said first part (1) of the connection module in the direction of the said second part (2) of the connection module and which coupling pin (4) is preferably oriented in the direction of mutual coupling and decoupling of the said two parts (1, 2), while
said second part (2) has a seat (5) for engaging the said coupling pin (4) which is in a position axially coinciding with the said coupling pin (4) and which has at least one hooking tooth (9) to the enlarged head (401) of the said coupling pin (4), which hooking tooth is movable along a coupling/uncoupling stroke alternatively in a position radially distanced from the perimeter of the said enlarged head (401) of the said coupling pin (4) and in a position radially close to the said coupling pin (4) and axially flanked by the radial shoulder formed by the said enlarged head (4) and behind the said radial shoulder with reference to the direction of engagement of the said coupling pin (4) in the said engagement seat(5) of the said second part (2),
the said hooking tooth being controlled to execute the said coupling/uncoupling stroke by the said control actuator member and by the said actuation member, or by the said pusher (8).

5. Module according to one or more of the previous claims, in which said first part (1) and said second part (2) have end-of-stroke abutment surfaces facing each other and which abutment surfaces present coinciding crowns of relative angular positioning notches (102, 122) of said first and second part (1, 2) between them.

6. Module according to one or more of the previous claims, wherein said connection module has a rotationally symmetrical shape, the direction of coupling and decoupling of said two parts (1, 2) being parallel to the central axis of the connection module.

7. Module according to claim 6, in which the axis of symmetry coincides with the axis of the coupling pin (4) and with the corresponding coupling seat (5) of the said second part (2), while the said crowns of reciprocal angular positioning notches (102, 122) are constituted by two annular crowns of concentric circles of axial teeth, preferably with a rounded or substantially sinusoidal profile.

8. Module according to claim 7, in which at least one of the said crowns of axial angular positioning notches (102, 122) is designed to be mountable and removable to and from at least one of the corresponding part (1, 2) of the connection module, optionally by means of an interlocking system of the type known as bayonet (121, 203).

9. Module according to one or more of the previous claims, in which the said hooking tooth is constituted by the perimeter edge of a circular hole (501) provided in a frame (9), which hole (501) has a diameter slightly greater than the major diameter of the enlarged head (401) of the coupling pin (4) and which frame (9) is mounted sliding in the direction of a diametral axis of the coupling hole (501), preferably parallel to at least one side of said frame (9), which axis is parallel with the sliding axis of said pusher (8) and which pusher controls the movement of the said frame (9) at least in one direction, while the movement in the opposite direction is determined by said elastic elements, and the movement of the frame (9) in the direction radially outwards from the pusher (8) is caused by said elastic means while the movement in the opposite direction is caused by said surface cam, and in which the said elastic elements bring the frame (9) into an eccentric position of the hole (501) with respect to the axis of the coupling pin (4) and in which a part of the perimeter edge of the said hole (501) is positioned behind the radial annular shoulder of the enlarged head (401) with reference to the direction of engagement of the coupling pin (4) in the said coupling seat (5) and while in the said position of the pusher (8) corresponding to the end of stroke having minor radial protrusion outwards from the said face of the said perimeter wall of the casing (201), the said frame (9) is brought by the said pusher (8) into a coaxial position with the said enlarged head (401) of the coupling pin (4).

10. Module according to one or more of the previous claims, wherein at least the enlarged head (401) of the coupling pin (4) has a rotationally symmetrical shape and is optionally conically tapered in the direction of the front end with reference to the direction of coupling of the said two module parts (1, 2) one to the other and/or of engagement of the coupling pin (4) into the corresponding coupling seat (5) .

11. Module according to one or more of the previous claims in which the said cam shaped surface is provided coinciding with a side wall sector (20, 21; 240, 340; 261, 262, 263; 281, 282, 283) of a parking cradle (30; 40; 61, 62, 63; 81, 82, 83) for a gripping tool and/or an end effector (31, 32; 41; 51, 52, 53; 71) which side wall sector is intended to overlap laterally on the perimeter wall of the casing of the second part (2) of the connection module and which wall sector is at least partially U-shaped or has two diametrically opposed sides which converge towards each other in the direction of the insertion end position of the gripping tool and/or of the end effector (31, 32; 41; 51, 52, 53; 71) and of the second module part (2) associated with them and while one of the said two lateral walls of the said U-shaped sector cooperates with the sliding head (801) of the pusher (8) and the other of the two lateral walls of the said U-shaped sector cooperates with the face of the perimeter wall of the mantle surface of the said second part (2) of the connection module which is diametrically opposite to the said sliding head (801) of the said pusher (8), and while the reduction of the distance between the said two lateral walls corresponds to the stroke of moving the pusher (8) in the radial direction of the coupling pin (4) and/or of the frame (9) with the coupling hole (5) with which said hooking hole (5) is brought into a coaxial position with the said coupling pin (4).

12. Module according to claim 11, wherein said parking cradle (30; 40; 61, 62, 63; 81, 82, 83) is constituted by a concave housing which in correspondence with one open side presents the said U-shaped wall sector which constitutes the cam-shaped surface.

13. Module according to claims 11 or 12, wherein the parking cradle (30; 40; 61, 62, 63; 81, 82, 83) is open on two opposite sides parallel to the cross-section plane of the U-shaped shaped wall and this is provided oriented with its axis in a substantially vertical direction.

14. Module according to one or more of the previous claims **characterized by** the fact of being provided in combination with two or more parking cradles (30; 40; 61, 62, 63; 81, 82, 83) separate or combined together in a single box (90) and two or more gripping tools and/or end effectors (31, 32; 41; 51, 52, 53; 71),
Which box is intended to accommodate in a respective cradle one of said two or more gripping tools or two or more ends effectors (31, 32; 41; 51, 52, 53; 71), each of the said gripping tools and/or end effectors being configured differently and for the execution of different actions or activities and which two or more gripping tools and/or which two or more end effectors (31, 32; 41; 51, 52, 53; 71) are each provided with a connection socket to a terminal coupling to a stump which is made as the said second part (2) of the said connection module and is identical for each of the two or more gripping tools and/or for each of the two or more end effectors (31, 32; 41; 51, 52, 53; 71) or for some of the said two or more gripping tools and/or end effectors, while the said connection socket is fixed or can be fixed to the said first part (1) of said connection module provided on the coupling terminal to a stump, being possible to replace the gripping tool and/or the end effector (31, 32; 41; 51, 52, 53; 71) connected to the stump coupling terminal with any of the gripping tools or end effectors (31, 32; 41; 51, 52, 53; 71) present in the respective cradles (30; 40; 61, 62, 63; 81, 82, 83) of the parking box (90).

15. Module according to one or more of the preceding claims in which it is provided in combination with at least one of the said gripping tools and/or end effectors (71) which is configured in the shape of a hand and which has at the end distal from the fingers a coupling module to a terminal for fixing to a stump, while said hand part is shaped similarly to a hand in gripping position for a gripping handle (72) having a longitudinal dimension preponderant with respect to a dimension transverse to said longitudinal direction and the position of the thumb being opposite to the position of the remaining fingers so as to form a housing channel, respectively being provided circumferential clamping members (73, 74) of a handle which are positioned at the two opposite lateral ends of the palm of the hand, said clamping members being elastically clampable and/or clampable by tensioning around a tool handle.

16. Module according to claim 15, wherein the clamping elements (73, 74) are in the form of a tape, a cable, a rope or a string, or the like which each form at least one loop and which present a pre-established prior tension and in in which the following configurations are provided alternatively or in combination with each other:
the preventive tension is such that the fixing of a handle occurs by forcing it when introducing it into the handle;
the preventive tension is such that it is further modified or regulated in relation to the clamping force by means of a pre-established force, a tensioning/loosening mechanism being provided in combination with at least some of the said clamping elements which can be operated manually or by means of an electric motor.

17. Module according to claims 15 or 16, in which the hand is made hollow at least in the area coinciding with the part of the back thereof and the cavity (75) is closed towards the outside by a removable cover (76) which is preferably shaped so as to complete the shape of the back of the hand in correspondence with the opening of the said cavity (75) and which has removable coupling elements (77) with corresponding and coinciding elements (78) provided in the said cavity and in which optionally the said cavity (75) constitutes the housing compartment of the terminal ends of the clamping members (73, 74) and/or housing the tensioning and loosening mechanism(s) of the said clamping members (73, 74), as well as the possible driving motor(s) of the said tensioning and loosening members and the related control circuits and/or the power batteries.

18. Gripping tool (71) **characterized by** the fact that it is configured in the shape of a hand and which has at the end distal from the fingers a coupling module to a terminal for fixing to a stump, while said hand part is shaped similarly to a hand in gripping position for a gripping handle (72) having a longitudinal dimension preponderant with respect to a dimension transverse to said longitudinal direction and the position of the thumb being opposite to the position of the remaining fingers so as to form a housing channel, respectively being provided circumferential clamping members (73, 74) of a handle which are positioned at the two opposite lateral ends of the palm of the hand, said clamping members being elastically clampable and/or clampable by tensioning around a tool handle.

19. Gripping tool (71) according to claim 18, wherein the clamping elements (73, 74) are in the form of a tape, a cable, a rope or a string, or the like which each form at least one loop and which present a pre-established prior tension and in in which the following configurations are provided alternatively or in combination with each other:
the preventive tension is such that the fixing of a handle occurs by forcing it when introducing it into the handle;
the preventive tension is such that it is further modified or regulated in relation to the clamping force by means of a pre-established force, a tensioning/loosening mechanism being provided in combination with at least some of the said clamping elements which can be operated manually or by means of an electric motor.

20. Gripping tool (71) according to claims 18 or 19, in which the hand is made hollow at least in the area coinciding with the part of the back thereof and the cavity (75) is closed towards the outside by a removable cover (76) which is preferably shaped so as to complete the shape of the back of the hand in correspondence with the opening of the said cavity (75) and which has removable coupling elements (77) with corresponding and coinciding elements (78) provided in the said cavity and in which optionally the said cavity (75) constitutes the housing compartment of the terminal ends of the clamping members (73, 74) and/or housing the tensioning and loosening mechanism(s) of the said clamping members (73, 74), as well as the possible driving motor(s) of the said tensioning and loosening members and the related control circuits and/or the power batteries.

21. Gripping tool (71) according to one or more of claims 18 to 20, which gripping tool (71) is fixable or stably presents a part (2) of a coupling module to a terminal for fixing to a stump, which part (2) and which coupling module are made according to one or more of the previous claims 1 to 15.
